Europäisches Patentamt

European Patent Office

Office européen des brevets

(11) Veröffentlichungsnummer: **0 304 689 B1**

(12) # EUROPÄISCHE PATENTSCHRIFT

(45) Veröffentlichungstag der Patentschrift: **28.10.92**

(21) Anmeldenummer: **88112615.5**

(22) Anmeldetag: **03.08.88**

(51) Int. Cl.5: **A61K 31/295**, A61K 33/06,
A61K 33/24, A61K 33/26,
A61B 17/22, //(A61K33/06,
31:295),(A61K33/26,33:24,
33:06)

(54) **Lösung zur Anwendung als Spülfüssigkeit in der Zerstörung von körperfremden Ablagerungen in menschlichen und tierischen Geweben oder Körperhohlräumen.**

(30) Priorität: **28.08.87 DE 3728814**

(43) Veröffentlichungstag der Anmeldung:
**01.03.89 Patentblatt 89/09**

(45) Bekanntmachung des Hinweises auf die
Patenterteilung:
**28.10.92 Patentblatt 92/44**

(84) Benannte Vertragsstaaten:
**AT BE CH DE ES FR GB GR IT LI LU NL SE**

(56) Entgegenhaltungen:
**DE-A- 2 252 665**
**DE-A- 3 213 139**

(73) Patentinhaber: **LEOPOLD PHARMA GESELL-
SCHAFT m.b.H.**
**Hafnerstrasse 36**
**A-8055 Graz(AT)**

(72) Erfinder: **Reichel, Erich, Dr.**
**Kopernikusgasse 11**
**A-8010 Graz(AT)**
Erfinder: **Schmidt-Kloiber, Heinz, Dr.**
**Grossgrabenweg 5 A**
**A-8010 Graz(AT)**
Erfinder: **Groke, Karl, Dr.**

**A-8063 Eggersdorf Nr. 327(AT)**

Rank Xerox (UK) Business Services

**Beschreibung**

Die vorliegende Erfindung betrifft eine Lösung, die zur Anwendung als Spülflüssigkeit in der Zerstörung von körperfremden Ablagerungen in menschlichen oder tierischen Geweben oder Körperhohlräumen dient, wobei diese Zerstörung durch Stoßwellen erfolgt, die in dieser Spülflüssigkeit durch einen laserinduzierten Durchbruch erzeugt werden.

Im Zuge von Untersuchungen, eine möglichst schonende Methode zur Zerstörung von körperfremden Ablagerungen wie z. B. Nierensteine, Gallensteine, Harnleitersteine oder verkalktes Gewebe, zu finden, fehlte es nicht an Vorschlägen und Versuchen, Lichtenergie anstelle von anderen Energieformen wie Ultraschall oder elektrische Energie zu diesem Zweck heranzuziehen.

Eine Möglichkeit besteht darin, gepulste Laserstrahlung direkt auf die Oberfläche des zu zerstörenden Konkrements zu applizieren und dieses damit auf thermischem Weg zu zerstören. Über einen erfolgreichen klinischen Versuch dieser Methode zur Zerstörung von Gallengangsteinen mittels eines blitzlampengepulsten (flashlamp-pulsed) Lichtes eines Neodym-YAG-Lasers wird in DMW 1986, 111, Nr. 31/32, Seite 1217 berichtet. Hier wurden Laserpulse einer Dauer von 2 ms über eine flexible, 0,2 mm dicke Glasfaser, welche in einem Spezialkatheter geführt wurde, an die Steine herangeführt, wobei gleichzeitig Wasser und Kontrastmittel instilliert wurden. Bei dieser thermischen Methode ist die benötigte Energiemenge hoch und die Umgebung des Operationsfeldes von einer thermischen Belastung kaum zu schützen.

Es wurde auch schon vorgeschlagen, das an der Oberfläche des Steins absorbierte Laserlicht für einen elektroakustischen Effekt auszunützen, was einen niedrigen Energiebedarf zur Folge hätte. Dazu ist Voraussetzung, Laserlicht einer Wellenlänge zu verwenden, das nur ein geringes Eindringungsvermögen in das Material des Steins besitzt. Dies trifft für Laserlicht im sichtbaren Bereich, etwa bei 350 bis 550 nm zu, wobei innerhalb dieses Bereiches die Wellenlänge an das Material des Konkrements anzupassen ist.

Eine Vorrichtung für die Durchführung dieser Methode ist in der WO 86/06269 beschrieben, bei der ein Laser vom Typ des sog. "dyelasers" zur Anwendung kommt. Neben dem Nachteil, die Wellenlänge des Laserlichtes an die chemische Natur des Konkrements anpassen zu müssen, um die Methode mit Erfolg durchführen zu können, besitzt diese Methode auch den weiteren Nachteil der Benützung von Laserlicht im sichtbaren Bereich, das zum Schutz der Augen des durchführenden Arztes starke Lichtfilter erforderlich macht, die die Beobachtung des Operationsfeldes ungemein erschweren.

Von Schmidt-Kloiber H. wurde in "Energieumwandler zur Steinzerstörung in den ableitenden Harnwegen des Menschen", Aktuelle Nephrologie 1 (1978), Seite 138 - 144 bereits vorgeschlagen, zu diesem Zweck eine Methode heranzuziehen, bei der Lichtenergie in unmittelbarer Nähe des Konkrements in mechanische Energie in Form einer Kavitationsblase, verbunden mit einem Auftreten von Stoßwellen, umgewandelt wird, wobei diese mechanische Energie für die Zerstörung des Konkrements verantwortlich ist. Zu diesem Zweck wird gepulstes Laserlicht über einen Lichtleiter in das Operationsgebiet gebracht und in Oberflächennähe, nicht jedoch an der Oberfläche des zu zerstörenden Steins konzentriert, wobei die dabei auftretende große elektrische Feldstärke in der durch laufende Spülung mit Spülflüssigkeit bestehenden flüssigen Umgebung des Steins zu sogenannten laserinduzierten Durchbrüchen führt, die Stoßwellen und Kavitation zur Folge haben, die die Zerstörung des Steins bewirken. Da die Steinzerlegung dabei durch die mechanische Energie der Stoßwellen bewirkt wird, besteht bei dieser Methode der Vorteil, daß deren Erfolg unabhängig von der chemischen Natur des Steins ist und damit auch alle anderen Nachteile der oben geschilderten Methoden vermieden werden. Die grundlegenen physikalischen und technischen Gesichtspunkte dieser Methode werden von H. Schmidt-Kloiber, E. Reichel und H. Schöffmann in Biomed. Technik 30 (1985), 173 - 181, beschrieben.

Wie von H. Schmidt-Kloiber und E. Reichel in Acustica Vol. 54 (1984), Seite 284, aufgezeigt wird, erfolgt in Flüssigkeiten nur bei großen Pulsenergien zu jeder Laseremission ein laserinduzierter Durchbruch. Mit abnehmender Laserpulsenergie nimmt die Häufigkeit der Durchbrüche ab, bis eine Schwellenenergie erreicht ist, nach deren Unterschreitung praktisch keine Durchbrüche mehr zu erzielen sind. Diese Ergebnisse sind von Bedeutung, da einerseits die Effektivität der Methode von einer guten Ausnützung der abgegebenen Laserpulse abhängt, andererseits aber die zu übertragende Intensität durch die Notwendigkeit der Benützung eines Lichtleiters begrenzt ist, da man die Intensität nicht so hoch wählen darf, daß bereits im Lichtleiter laserinduzierte Durchbrüche auftreten.

Durch die oben zitierte Arbeit von Schmidt-Kloiber H. und Reichel E. in Acustica Vol. 54 ist auch schon bekannt geworden, daß bei in vitro Testungen, die mit Wasser, einer wäßrigen Lösung von 27 g Sorbit und 5,4 g Mannit pro Liter und einer 0,9%igen Kochsalzlösung durchgeführt wurden, die Schwellenergie bei Verwendung von Kochsalzlösung am niedrigsten war und bei dieser Lösung die Durchbruchshäufigkeit mit steigender Pulsenergie rascher zunahm als bei den beiden anderen getesteten Flüssigkeiten. Diese Ergebnisse wurden mit einer Versuchsanordnung erhalten, bei der das gepulste Laserlicht über eine Sammellinse in die Küvette fokussiert wurde, die die Versuchsflüssigkeit enthielt. Für eine in vivo Anwendung der Methode der Steinzerstörung ist allerdings zu berücksichtigen, daß der Lichttransport durch einen optischen Leiter einen Energieverlust bedeutet, sodaß mehr Energie anzubieten ist, als für eine gemäß dem oben geschilderten in vitro Test ausreichende Durchbruchshäufigkeit erforderlich ist. Hierbei sind aber Grenzen gesetzt, da nicht nur die Schwellintensität für einen Durchbruch im Lichtleitermaterial nicht überschritten werden darf, sondern auch der Durchmesser des Lichtleiters aus Gründen der Verwendbarkeit im Körper mit etwa 1 mm begrenzt ist (Biomedizinische Technik 30 (1985), Seite 177).

Praxisnahe Tests unter Verwendung von Lichtleitern, die im Rahmen von Versuchen durchgeführt wurden, die zur vorliegenden Erfindung führten, zeigen, daß die Schwellenenergie von 0,9%iger Kochsalzlösung, vor allem bei Anwendung von im Infrarotbereich liegendem Laserlicht, immer noch zu hoch liegt, um eine brauchbare Durchbruchshäufigkeit zu erzielen. Es bestand daher die Aufgabe, flüssige Medien zu finden, mit denen in physiologisch verträglichen Konzentrationen bei Verwendung des Lichtleiters eine große Durchbruchshäufigkeit erzielt wird. Da der Durchbruch mit der Ausbildung eines hell leuchtenden Plasmas (Gasblase) verbunden ist, kann dieser auch visuell erkannt und die daraus resultierende Stoßwelle (Wirkung) qualitativ beurteilt werden.

Überraschenderweise konnte nun gefunden werden, daß Verbindungen der Eisenmetalle Eisen, Kobalt und Nickel, sowie der Erdalkalimetalle Magnesium und Calcium, wenn sie in einer wäßrigen Lösung vorliegen, die nur diese als wirksame Metallverbindungen enthält, in ungleich geringeren Konzentrationen als für Natriumchlorid eine wesentlich niedrigere Schwellenenergie besitzen als dieses und daß diese Verbindungen in diesen Lösungen in besonderer Weise zur Elektronengeneration befähigt sind, die sich in intensiver Plasmabildung, erkenntlich an einem intensiven Plasmaleuchten, äußert. Dieses Plasmaleuchten wird mit 0,9%iger Kochsalzlösung unter gleichen Versuchsbedingungen überhaupt nicht erhalten.

Aus der DE-A 32 13 139 ist bekannt geworden, daß der Zusatz unter anderem von Eisen- oder Magnesiumsalzen als Calcium komplexierende oder Oxalat komplexierende Verbindungen bei oraler Gabe erzielbare litholytische Wirkung von Carbonsäuren oder Carbonsäurederivaten auf Harnsteine verstärken, also eine chemische Zerstörung von Harnsteinen durch einen im Harn erzeugten Spiegel an diesen Carbonsäuren zu unterstützen vermögen. Im Gegensatz dazu haben die Lösungen gemäß vorliegender Erfindung, die Metallverbindungen aber keine litholytischen Komponenten enthalten, keine direkte Wirkung auf die Konkremente selbst, sondern dienen lediglich als Medium für die Erzeugung eines laserinduzierten Durchbruchs, wobei die dabei freiwerdende mechanische Energie die Steinzerstörung bewirkt. Die hervorragende Eignung der erfindungsgemäßen Lösungen, die nur lösliche Verbindungen der Eisenmetalle des Periodischen Systems und/oder der Erdalkalimetalle Magnesium oder Calcium als wirksame Verbindungen in einer Konzentration von maximal 50 mmol/Liter enthalten, für diesen Zweck, wird durch die DE-A 32 13 139 in keiner Weise beschrieben oder auch nur nahegelegt.

Gegenstand der vorliegenden Erfindung ist demnach eine Lösung zur Anwendung als Spülflüssigkeit, in der bei der Zerstörung von körperfremden Ablagerungen in menschlichen oder tierischen Geweben oder Körperhohlräumen mittels intensiver, gepulster Laserstrahlung, insbesondere solcher im Infrarotbereich, durch einen laserinduzierten Durchbruch die Zerstörung bewirkende Stoßwellen und Kavitation erzeugt werden, die dadurch gekennzeichnet ist, daß sie aus einer wäßrigen Lösung von Salzen oder Komplexverbindungen von Metallen der Eisengruppe des Periodischen Systems, von Magnesium oder Calcium oder von Mischungen solcher Salze oder Komplexverbindungen, die in einer Konzentration von maximal 50 mmol/Liter zugegen sind, besteht, die gegebenenfalls durch physiologisch verträgliche, inerte Zusätze auf eine Osmolarität, die etwa im Bereich jener einer physiologischen Kochsalzlösung liegt, eingestellt ist.

Als Metalle der Eisengruppe kommen Kobalt, Mangan und Nickel in zweiwertiger Form sowie zwei- als auch dreiwertiges Eisen in Frage.

Da die Methode der Steinzerstörung mittels Laserstrahlen (Laserlithotripsie) von der chemischen Natur des Konkrements unabhängig ist, ist diese Methode keinesfalls auf spezielle Konkremente wie Harnleitersteine, Nierensteine oder Gallensteine beschränkt, sondern kann für die Zerstörung jeglicher Ablagerungen in biologischen Materialien, Geweben, Blutgefäßen oder Körperhohlräumen herangezogen werden, zumal der Vorteil besteht, daß eine thermische Belastung des umgebenden Gewebes praktisch auszuschließen ist. Bei der Auswahl der Konzentration der Metallverbindungen oder -komplexe sind naturgemäß außer der Wirksamkeit auch die physiologischen Verhältnisse am Anwendungsort zu berücksichtigen.

Wird die Zerstörung von Konkrementen in lebenden Organismen, insbesondere am Menschen durchgeführt, empfiehlt es sich, den pH-Wert der erfindungsgemäßen Lösung auf 4-8, in besonders empfindlichen Gebieten sogar auf 4,5 - 6,5, einzustellen.

Von großer Bedeutung ist, daß die im Sinne der vorliegenden Erfindung wirksamen Konzentrationen der in der erfindungsgemäßen Lösung enthaltenen Stoffe weit unter den Konzentration der bisher empfohlenen 0,9%igen Kochsalzlösung liegen, die ja 0,15 mol/Liter entspricht. Durch diese sehr niedrigen wirksamen Konzentrationen können viel leichter physiologisch verträgliche Lösungen hergestellt werden. Die wirksamen Konzentrationen der einzelnen erfinungsgemäßen Metallsalze und Metallverbindungen sind unterschiedlich, liegen aber in der Regel zwischen 0,015 und 50 mmol/Liter. Es zeigte sich auch, daß jede der erfindungsgemäßen Lösungen in Abhängigkeit von der Konzentration ein Minimum in der Schwellenergie besitzt. Ein solches Phänomen wurde auch bei wäßriger Kochsalzlösung festgestellt (Schmidt-Kloiber und Reichel, Acustia 54 (1984), Seite 287), doch liegt hier das Minimum nicht im Bereich einer Konzentration von einigen Millimolen oder gar von Bruchteilen von Millimolen pro Liter, sondern bei etwa 0,5 bis 1 mol/Liter, einem Konzentrationsbereich, der aus physiologischen Gründen gar nicht mehr ausgenützt werden kann.

Überraschenderweise zeigte sich auch, daß bei Veränderung der Konzentration nicht nur höhere Schwellfeldstärken notwendig sind, sondern auch die Größe der entstehenden Bruchstücke verändert wird. Dies kann bei unterschiedlichen Anwendungen von Vorteil sein, da je nach Wahl der wirksamen Verbindung und/oder deren Konzentration in der Lösung entweder grobe Bruchstücke entstehen oder aber die Konkremente zu grießartigen oder pulverförmigen Teilen abgebaut werden. Prinzipiell ist dabei festzuhalten, daß Größe der Bruchstücke und Zerstörungszeit miteinander korrelieren. Man wird daher sowohl die Wahl der wirksamen Verbindung als auch deren Konzentration innerhalb der erfindungsgemäßen Grenzen nach dem Anwendungsfall richten, je nachdem ob Zerstörung in möglichst kurzer Zeit oder aber Zerstörung zu möglichst feinen Teilchen erwünscht ist oder ein Mittelweg zu beschreiten ist. Die jeweils optimale Zusammensetzung kann durch einige wenige Tests ermittelt werden. Allgemein gilt jedoch, daß geringere Konzentrationen der Metallverbindungen bzw. - komplexe die Zerlegung der Konkremente zu pulverförmigen oder grießartigen Teilen begünstigen, während mit ansteigender Konzentration die Tendenz zur Zerlegung des Konkrements zu gröberen Partikeln wächst.

Wie schon erwähnt, sind die optimalen Konzentrationen der erfindungsgemäß zu wählenden Metallverbindungen in unterschiedlichen Konzentrationsbereichen gelegen. Lösungen, die als wirksames Agens Verbindungen von Calcium oder Magnesium enthalten, in denen diese beiden Metalle zweckmäßig als lösliche Salze, beispielsweise als Chloride, vorliegen , werden bevorzugt in einer Konzentration von 1 bis 50 mmol/Liter angewendet. Hierbei ist Magnesium bevorzugt, da es trotz etwa gleicher Schwellenergie wie Calcium eine bessere Plasmabildung, erkennbar an einem kontinuierlichen Plasmaleuchten, bewirkt. Damit wird bei Verwendung von Magnesiumverbindungen in der Spüllösung mehr mechanische Energie freigesetzt, als bei der Verwendung von Calciumverbindungen in der gleichen Konzentration. Salze von Kobalt, Nickel und zweiwertigem Eisen werden bevorzugt in einem Konzentrationsbereich von 1 bis 5 mmol/Liter eingesetzt, wobei im Falle von Kobalt und Nickel die physiologische Verträglichkeit am Anwendungsort bei der Wahl der Konzentration innerhalb des bevorzugten Bereiches zu berücksichtigen ist. Auch die Verbindungen dieser Ionen erreichen trotz günstiger Lage der Schwellenergie die Magnesiumverbindungen in der Intensität der Plasmabildung nicht, bieten aber auch in diesem Punkt Vorteile gegenüber 0,9%iger Kochsalzlösung.

Eine Sonderstellung nehmen überraschenderweise die Verbindungen des dreiwertigen Eisens ein, die die anderen erfindungsgemäß zu wählenden Metallverbindungen, auch jene des zweiwertigen Eisens, sowohl in der Lage der Schwellenergie als auch in der Intensität der Plasmabildung bei weitem übertreffen.

Manche Salze oder Komplexverbindungen zeigen eine optimale Wirkung bereits bei Konzentrationen, die weit unter den wirksamen Konzentrationen der anderen erfindungsgemäß zu wählenden Metallverbindungen liegen. So zeigte beispielsweise eine wäßrige Lösung, die nur 0,05 mmol/l dreiwertiges Eisen in Form eines Eisen-Dextran-Komplexes enthielt, eine Schwellenergie, die nur etwa 1/5 jener von 0,9%iger Kochsalzlösung betrug, wobei die Intensität des Plasmaleuchtens jene von Lösungen aller anderen Metallverbindungen übertraf. Wählt man den gleichen Fe-III-Komplex in einer Konzentration von 0,5 mmol/l, so ist die Schwellenergie so niedrig, daß sie mit der gewählten Anordnung nicht mehr bestimmbar ist.

Auch dreiwertiges Eisen kann in der erfindungsgemäßen Lösung als einfaches Salz, z. B. als Eisentrichlorid, enthalten sein. Aus Gründen der einfachen Handhabung und der Haltbarkeit der Lösungen ist es jedoch empfehlenswert, das dreiwertige Eisen in Form eines seiner Komplexe einzusetzen. Hiezu kann jeder Komplex herangezogen werden, der das dreiwertige Eisen nur so fest gebunden enthält, daß noch Fe-III-Ionen an die Lösung abgegeben werden. Besonders bevorzugt sind der Eisencitrat-Komplex, dessen bevorzugte Konzentration im Bereich von 1 bis 5 mmol/Liter liegt, der Eisen-Tartrat-Komplex, dessen bevorzugte Konzentration 0,015 bis 1 mmol/Liter beträgt und die Eisen-Dextran-Komplexe, beispielsweise solche mit einem Eisengehalt von 10 bis über 30 %, einer Molmasse des Dextrans von 2000 - 6000 und eines Dextrananteiles von bis zu 50 %. Die optimal zu wählende Konzentration liegt bei letzteren ebenfalls im Bereich von 0,015 bis 1 mmol/Liter, bezogen auf den Eisengehalt. Derartige Eisen-Dextrankomplexe sind zum Beispiel in der DE-B-30 26 868 und in der DE-A-34 22 249 beschrieben. Als ebenso geeignet erweisen sich in der pharmazeutischen Praxis gängige eisenhaltige Komplexe, wie Eisen-Dextrine oder Eisensaccharat. Auch diese werden bevorzugt in einer Konzentration von 0,015 bis 1 mmol Eisen pro Liter eingesetzt.

Da dreiwertiges Eisen bereits in extrem niedrigen Konzentrationen seine Wirksamkeit im Sinne der vorliegenden Erfindung entfaltet, ist es auch möglich, in wäßriger Lösung Salze derselben in situ zu erzeugen, indem elementares Eisen, z. B. in Form von Pulver oder als Streifen in die Lösung eingebracht wird, wobei sich die anfängliche Konzentration an Fe-III-Salzen durch die Einwirkung der Laserstrahlung zunehmend erhöht.

Bei Anwendung der erfindungsgemäßen Lösung in der Laserlithotripsie ist es häufig aus physiologischen Gründen empfehlenswert, daß die Osmolarität der Lösungen etwa im Bereich einer physiologischen Kochsalzlösung liegt. Da die wirksamen Konzentrationsbereiche der erfindungsgemäßen Lösungen viel zu niedrig liegen, um isoton zu sein, ist es vorteilhaft, die Osmolarität durch Zusatz von Substanzen einzustellen, die physiologisch unbedenklich sind und mit den wirksamen Metallverbindungen in keiner Weise reagieren. Als solche Substanzen können z.B. Kohlenhydrate, insbesondere Sorbit oder Mannit oder Mischungen derselben oder auch Kochsalz dienen, wobei Kochsalz zur Einstellung der Osmolarität bevorzugt ist. Auf die Schwellenergie der Lösungen und die Plasmabildung haben diese Zusätze praktisch keinen Einfluß, wie Untersuchungen gezeigt haben.

Die Herstellung der erfindungsgemäßen Lösungen gelingt auf übliche Weise durch Auflösen der Metallsalze oder -komplexe in Wasser oder aber in einer Lösung physiologisch verträglicher, inerter Substanzen, deren Konzentration so zu wählen ist, daß sie zusammen mit den wirksamen Bestandteilen etwa eine isotone Lösung ergeben. Anschließend wird, wenn nötig der pH-Wert eingestellt und die Lösung steril gemacht, wozu je nach Beständigkeit der wirksamen Substanz sowohl eine Hitzesterilisation als auch eine Sterilfiltration geeignet sind. Es ist auch möglich, die erfindungsgemäße Lösung erst unmittelbar vor dem Gebrauch zusammenzumischen. Zu diesem Zweck wird eine entsprechende Menge einer konzentrierten Salzlösung oder einer konzentrierten Lösung eines Komplexes in steriler Form, z. B. in einer Ampulle bereitgestellt, die dann unmittelbar vor Gebrauch der gewünschten Menge an sterilem Wasser oder einer Lösung entsprechender Osmolarität zugesetzt und dort gleichmäßig verteilt wird. Ebenso kann aber das Metallsalz oder die Komplexverbindung in fester Form, z. B. als Lyophilisat, bereitgestellt werden, das dann unmittelbar vor Gebrauch in einer kleinen Menge sterilem Wasser aufgenommen wird. Die so resultierende Lösung wird dann auf das endgültige Volumen aufgefüllt. Die Vorgangsweise der Bereitung an Ort und Stelle bietet sich vor allem dort an, wo das gewählte Salz oder die Komplexverbindung in wäßriger Lösung instabil ist und beispielsweise in verdünnter Lösung einer Hitzebehandlung, wie sie zur Sterilisation nötig ist, nicht standhält. Die Bereitung an Ort und Stelle kann aber auch in Fällen günstig sein, wo man für alle möglichen Zwecke Wasser ad injectionem oder physiologische Kochsalzlösung zur Verfügung hat und die Lagerung der erfindungsgemäßen Lösung in anwendungsfertiger Form aus Platzgründen nicht erwünscht ist, oder ein Transport über weite Entfernungen erforderlich ist. Diese Vorgangsweise empfiehlt sich vor allem dann, wenn Salze oder Komplexe von dreiwertigem Eisen als wirksames Agens dienen sollen, da verdünnte Lösungen von dreiwertigem Eisen Probleme bei der Haltbarkeit mit sich bringen können und außerdem Eisen-III-Verbindungen in so geringer Konzentration eingesetzt werden, daß auch dann, wenn isotone Lösungen erwünscht sind, als Basislösung physiologische Kochsalzlösung herangezogen werden kann, die in jedem Spital vorhanden ist, die dann keiner Konzentrationseinstellung vor der Mischung bedarf.

Die erfindungsgemäße Lösung ist als Spüllösung in allen Varianten der Laserlithotripsie, bei denen die Steinzerstörung oder Zerstörung sonstiger fester Ablagerungen durch Stoßwellen erfolgt, die in deren flüssiger Umgebung erzeugt werden, einsetzbar, gleichgültig, auf welche Weise die Strahlung im Operationsfeld konzentriert wird. Dabei kann mit Laserlicht verschiedener Wellenlängen gearbeitet werden, bevorzugt wird jedoch Laserlicht eingesetzt, dessen Wellenlänge im Infrarotbereich liegt, da dadurch bei Anwendung des notwendigen Augenschutzes die visuelle Wahrnehmung durch den Arzt nicht eingeschränkt wird und der Effekt der erfindungsgemäßen Lösung bei Verwendung von Laserlicht im Infrarotbereich besonders ausgeprägt ist. Schließlich sind Lasersysteme, die im Infrarot arbeiten, seit Jahren als äußerst zuverlässig und einfach bedienbar bekannt. Dies gilt besonders für Neodym-YAG-Laser. Im Hinblick auf die niedere Schwellenergie dieser Lösungen, insbesondere dann, wenn es sich um Lösungen mit dreiwertigem Eisen als wirksames Agens handelt, sind diese auch dort anwendbar, wo aus physiologischen oder apparativen Gründen auf eine besonders starke Bündelung der eingesetzten Laserstrahlen verzichtet werden muß, oder aus anderen Gründen längere als die bisher beschriebenen Laserpulse verwendet werden.

In den nachfolgenden Beispielen werden einige besonders bewährte Rezepturen für erfindungsgemäße Lösungen angegeben. Ferner werden Meßwerte, die mit den erfindungsgemäßen Lösungen erhalten wurden, angegeben.

Da die Schwellenergie von der Art und der Wellenlänge des Laserstrahles abhängig ist und in die Meßwerte auch geometrische Daten des Laserstrahles eingehen, können Zahlenwerte, die mit verschiedenen Einrichtungen erhalten wurden, nicht miteinander verglichen werden. Daher wurde als objektives Bewertungskriterium der Vergleich mit 0,9%iger Kochsalzlösung gewählt, die in jeder Versuchsreihe als Standard mitgeführt wird. Die Schwellenergie von 0,9%iger Kochsalzlösung wird mit 100 % festgesetzt und die der Versuchslösung in Prozenten bezogen auf die 0,9%ige Kochsalzlösung angegeben. Als Energie der Laserstrahlung, die für die Messungen eingesetzt wurde, wurde jene gewählt, die bei 0,9%iger Kochsalzlösung gerade ausreicht, um eine Durchbruchshäufigkeit von 80 bis 100 % zu erzielen.

Zur weiteren Bewertung dienten Versuche, bei denen zum Lichttransport des Laserstrahles ein Lichtleiter herangezogen wurde, der in einer Küvette endet, die mit der Versuchsflüssigkeit gefüllt ist. Der Lichtleiter wurde eintrittsseitig mit dem gepulsten Licht eines Güte-geschalteten Neodym-YAG-Lasers mit einer Energie von 39 mJ beaufschlagt. Die Pulsfolgefrequenz betrug 50 Hertz. Die Energie der Laserstrahlung an der Austrittsseite betrug 36 mJ. Als Kriterium für die Wirkung der Versuchsflüssigkeit wurde einerseits das Auftreten bzw. die Intensität des Plasmaleuchtens visuell beurteilt, andererseits wurde in die Küvette Steinmaterial mit leichter Fixierung in die Nähe der Austrittsseite des Lichtleiters gebracht und die Zerlegung wurde ebenfalls visuell beurteilt. Einige solcher Versuchsergebnisse sind in der Tabelle in Beispiel 9 sowie in Beispiel 10 angegeben.

Beispiel 1:

50 mmol Magnesiumchlorid werden in etwas weniger als 1 l Lösung aufgelöst, die 80 mmol Kochsalz in Wasser gelöst enthält. Nach erfolgter Auflösung und Auffüllen auf ein Volumen von 1 Liter wird eine isotone Lösung erhalten, die durch Hitzesterilisation bei 110 °C sterilisiert wird. Sie kann mit gutem Erfolg zur Zerstörung von Harnleitersteinen eingesetzt werden.

Beispiel 2:

50 mmol Calciumchlorid werden in 800 ml einer wäßrigen Lösung von 80 mmol Kochsalz aufgelöst, worauf mit Wasser ad injectionem auf 1 Liter eingestellt wird. Die Lösung ist nach Hitzesterilisation für die Zerstörung von Harnleitersteinen geeignet.

Beispiel 3:

In 900 ml einer wäßrigen Lösung von 154 mmol NaCl wird 1 mmol Fe-III-Citrat aufgelöst. Nach Einstellen des pH-Wertes mit Natronlauge auf pH 4,5 wird die Lösung auf 1 Liter aufgefüllt. Sie wird 30 Minuten bei 112 °C sterilisiert.

Diese Lösung ist physiologisch hervorragend verträglich und sowohl für den Einsatz in der Harnleitersonde als auch in einer Sonde, die durch Punktierung in den Körper eingeführt wird, geeignet.

Beispiel 4:

Wie in Beispiel 3 beschrieben, wird eine Lösung bereitet, die 1 mmol Fe-III/Liter, jedoch als Tartrat enthält. Sie kann ebenso wie die Lösung gemäß Beispiel 3 eingesetzt werden.

Beispiel 5:

Wie in Beispiel 3 beschrieben, wird eine Lösung mit einem Fe-III-Gehalt von 0.02 mmol/l hergestellt, wobei als Wirkstoff ein Eisen-III-Dextran-Komplex mit einem Eisengehalt von 16,0 % diente, in dem das Dextran einen Anteil von 15 bis 25 % besaß. Die Lösung wird 60 Minuten bei 113 °C sterilisiert. Sie kann trotz niedrigerer Konzentration als jene gemäß Beispiel 3 genauso wie jene Lösung eingesetzt werden.

Beispiel 6:

In der isotonen Lösung einer Mischung von 27 g Sorbit und 5,4 g Xylit pro Liter wird soviel des Fe-III-Dextran-Komplexes, wie es in Beispiel 5 verwendet wird, aufgelöst, daß die Lösung 0,02 mmol Fe-III pro Liter enthält. Sie wird sterilfiltriert und kann genauso eingesetzt werden wie die Lösung gemäß Beispiel 5.

Beispiel 7:

Aus dem gleichen Eisen-Dextran-Komplex wie in Beispiel 5 wird eine wäßrige Lösung mit einem Fe-III-Gehalt von 20 mmol/Liter hergestellt. Diese Lösung wird in 5 ml Ampullen abgefüllt und hitzesterilisiert. Vor dem Einsatz als Spüllösung wird der Inhalt der Ampulle in 4,5 Liter 0,9%iger Kochsalzlösung gleichmäßig verteilt.

Beispiel 8:

1 mmol $CoCl_2$ wird in etwas weniger als 1 Liter einer Lösung von 154 mmol NaCl aufgelöst und wie in Beispiel 3 beschrieben weiterbehandelt. Sie kann als Spüllösung bei der Zerstörung von Blasensteinen dienen.

Beispiel 9:

Von verschiedenen erfindungsgemäßen Lösungen wurden deren Eigenschaften im Vergleich zu 0,9%iger NaCl-Lösung bestimmt, wobei wie vorher geschildert, ohne und mit Lichttransport durch einen Lichtleiter gearbeitet wird.

Für die Bestimmung der Schwellenergie wurde folgendermaßen vorgegangen: Laserpulse mit einer Wellenlänge von 1064 nm und 8 ns Dauer wurden durch eine Sammellinse fokussiert. Eine Küvette wurde so einschiebbar angebracht, daß der Brennpunkt im Innenraum zu liegen kommt. Die Küvette wurde mit 0,9%iger Kochsalzlösung befüllt, und es wurde die Laserpulsenergie so eingestellt, daß bei dieser Kochsalzlösung gerade Durchbrüche mit einer Häufigkeit von 80 bis 100 % erzielt wurden. Dann wurde der zeitliche Verlauf des Laserpulses ohne Küvette und anschließend mit eingeschobener Küvette, die die jeweilige Versuchsflüssigkeit enthielt, registriert. Ein Durchbruch ist daran zu erkennen, daß das durchtretende Laserlicht plötzlich abbricht. Je führer dieser Abbruch eintritt, desto niedriger liegt die Schwellenergie.Der Zahlenwert der Schwellenergie errechnet sich aus der Fläche des ursprünglichen Laserpulses bis zur Stelle des Abbruches. Die erhaltenen Schwellenergien in mJ sind in der nachfolgenden Tabelle angegeben. Die Energie des Laserpulses in dieser Versuchsreihe betrug vor der Küvette 6,2 mJ, die Energie am Brennfleck 3,8 mJ. Zu jeder Versuchslösung wurde ferner die Schwellenergie in Prozenten, bezogen auf NaCl = 100 % berechnet.

Für die Untersuchungen, bei denen das Licht zuerst durch einen Lichtleiter mit 0,6 mm Durchmesser hindurchtritt, betrug die Energie am Austrittsende der Faser 36 mJ mit der Pulswiederholrate von 50 Hz. Für die optische Bewertung des Plasmaleuchtens diente folgende Beurteilungsskala:

0       kein visuell feststellbarer Durchbruch
1       sporadisches Auftreten von Durchbrüchen, erkennbar an aufblitzendem Plasmaleuchten
2       "kontinuierliches" Auftreten des Plasmaleuchtens
3       starkes "kontinuierliches" Auftreten von räumlich ausgedehntem Plasmaleuchten

Wellenlänge und Pulsdauer des verwendeten Laserlichtes waren gleich wie bei den Versuchen ohne optischen Lichtleiter.

Als Testlösungen wurden solche der wirksamen Verbindungen in bidestilliertem Wasser als Lösungmittel sowie solche, die durch Zusatz von NaCl isoton gemacht wurden, eingesetzt. Die Ergebnisse sind in der folgenden Tabelle zusammengefaßt.

Tabelle

| Versuch Nr. | Verbindung | Konz. mmol/l (Metall) | LM | ES mJ | ES/$E_{NaCl}$ % | LIB |
|---|---|---|---|---|---|---|
| Standard | NaCl 0,9 % | | | 1,52 | 100 | 0 |
| 1 | $FeCl_3$ | 0,2 | $H_2O$ | 0,10 | 6,58 | - |
| 2 | $FeCl_3$ | 0,1 | $H_2O$ | 0,10 | 6,58 | - |
| 3 | $FeCl_3$ | 0,04 | $H_2O$ | 0,10 | 6,58 | - |
| 4 | $FeCl_3$ | 0,2 | NaCl | 0,10 | 6,58 | - |
| 5 | $FeCl_3$ | 0,1 | NaCl | 0,10 | 6,58 | - |
| 6 | $FeCl_3$ | 0,04 | NaCl | 0,10 | 6,58 | - |
| 7 | Fe-Citrat | 5 | NaCl | 0,51 | 33,56 | 3 |
| 8 | Fe-Citrat | 1 | NaCl | 0,47 | 30,92 | 1 |
| 9 | Fe-Tartrat | 0,9 | NaCl | 0,59 | 38,82 | 3 |
| 10 | Fe-Tartrat | 0,1 | NaCl | 0,61 | 40,13 | 2,5 |
| 11 | Fe-Dextran | 0,9 | NaCl | 0,0 | 0 | 3 |
| 12 | Fe-Dextran | 0,5 | NaCl | 0,0 | 0 | 3 |
| 13 | Fe-Dextran | 0,02 | NaCl | 0,37 | 24,35 | 3 |
| 14 | $CoCl_2$ | 5 | NaCl | 0,97 | 63,82 | 2 |
| 15 | $CoCl_2$ | 1 | NaCl | 0,60 | 39,48 | - |
| 16 | $NiCl_2$ | 1 | NaCl | 0,96 | 63,17 | 1 |
| 17 | $MgCl_2$ | 50 | NaCl | 1,17 | 76,99 | 2 |
| 18 | $MgCl_2$ | 1 | NaCl | 0,52 | 34,22 | 1 |
| 19 | $CaCl_2$ | 50 | NaCl | 1,14 | 75,01 | 1 |
| 20 | $CaCl_2$ | 1 | NaCl | 0,78 | 51,32 | - |

Legende:

LM = Lösungsmittel

$H_2O$ = bidestilliertes Wasser

NaCl = Lösung, die mit NaCL isoton gestellt wurde ES = Schwellenergie der einzelnen Lösungen

$E_{NaCl}$ = Schwellenergie einer 0,9%igen NaCl-Lösung LIB = Bewertungsnote des Plasmaleuchtens

Wie die Tabelle erkennen läßt, gehen überraschenderweise die Werte für die Schwellenergie und die Intensität der Plasmabildung nicht völlig konform, was erkennen läßt, daß offenbar für die Erzielung der erfindungsgemäßen Wirkung auch noch andere Kriterien außer der Höhe der Schwellenergie mitspielen.

Beispiel 10:

In einer weiteren Testanordnung wurde das Laserlicht durch einen Lichtleiter in eine Küvette, die mit Lösungen eines Eisen-Dextran-Komplexes mit einer Konzentration von 0,1 mmol/l befüllt war, geleitet, wobei die Lösung 1 den Eisenkomplex in bidestiliertem Wasser, die Lösung 2 in 0,9%iger Kochsalzlösung und die Lösung 3 ihn in einer Lösung von 27 g Sorbit und 5,4 g Mannit pro Liter gelöst enthält.

In der Küvette wurde ein Harnsäurestein leicht fixiert. Der Stein wurde durch die angebotenen Laserpulse gleicher Energie wie in Beispiel 9 in den Versuchen mit Lichtleiter zur grießartigem Material abgebaut. Ein Unterschied in der Effektivität der Steinzerstörung zwischen den drei Lösungen war nicht zu beobachten.

**Patentansprüche**
**Patentansprüche für folgende Vertragsstaaten : AT, BE, CH, DE, FR, GB, IT, LI, LU, NL, SE**

1. Lösung zur Anwendung als Spülflüssigkeit, in der bei der Zerstörung von körperfremden Ablagerungen in menschlichen oder tierischen Geweben oder Körperhohlräumen mittels intensiver, gepulster Laserstrahlung, insbesondere solcher im Infrarotbereich, durch einen laserinduzierten Durchbruch die Zerstörung bewirkende Stoßwellen und Kavitation erzeugt werden, dadurch gekennzeichnet, daß sie aus einer wäßrigen Lösung von Salzen oder Komplexverbindungen von Metallen der Eisengruppe des Periodi-

8

schen Systems, von Magnesium oder Calcium oder von Mischungen solcher Salze oder Komplexverbindungen, die in einer Konzentration von maximal 50 mmol/Liter zugegen sind, besteht, die gegebenenfalls durch physiologisch verträgliche, inerte Zusätze auf eine Osmolarität, die etwa im Bereich jener einer physiologischen Kochsalzlösung liegt, eingestellt ist.

2. Lösung nach Anspruch 1, dadurch gekennzeichnet, daß zur Einstellung der Osmolarität Kochsalz dient.

3. Lösung nach Anspruch 1 oder 2, dadurch gekennzeichnet, daß der pH-Wert derselben bei 4 bis 8 liegt.

4. Lösung nach einem der Ansprüche 1 bis 3, dadurch gekennzeichnet, daß als Komplexverbindung von Metallen der Eisengruppe solche des dreiwertigen Eisens dienen.

5. Lösung nach Anspruch 4, dadurch gekennzeichnet, daß sie dreiwertiges Eisen als Citratkomplex in einer Konzentration von 1 bis 5 mmol/Liter enthält.

6. Lösung nach Anspruch 4, dadurch gekennzeichnet, daß sie dreiwertiges Eisen als Tartratkomplex in einer Konzentration von 0,015 bis 1 mmol/Liter enthält.

7. Lösung nach Anspruch 4, dadurch gekennzeichnet, daß sie dreiwertiges Eisen in Form eines Eisen-Dextran-Komplexes in einer Fe-III-Konzentration von 0,015 bis 1 mmol/Liter enthält.

8. Lösung nach einem der Ansprüche 1 bis 3, dadurch gekennzeichnet, daß ein lösliches Salz von Magnesium in einer Konzentration von 1 bis 50 mmol/Liter enthalten ist.

**Patentansprüche für folgende Vertragsstaaten : ES, GR**

1. Verwendung von Salzen oder Komplexverbindungen von Metallen der Eisengruppe des Periodischen Systems, von Magnesium oder Calcium oder Mischungen solcher Salze oder Komplexverbindungen zur Herstellung einer wäßrigen, nur diese Salze oder Komplexverbindungen als wirksame Metallverbindungen enthaltenden Spülflüssigkeit für die Zerstörung von körperfremden Ablagerungen in menschlichen oder tierischen Geweben oder Körperhohlräumen mittels Stoßwellen und Kavitation, die in dieser Spülflüssigkeit durch intensive, gepulste Laserstrahlung, insbesondere solche im Infrarotbereich, über einen laserinduzierten Durchbruch erzeugt werden, wobei diese Salze oder Komplexverbindungen zu einer Lösung einer Konzentration von maximal 50 mmol/Liter gelöst werden, die gegebenenfalls durch physiologisch verträgliche, inerte Zusätze auf eine Osmolarität eingestellt wird, die etwa jener einer physiologischen Kochsalzlösung entspricht.

2. Verwendung nach Anspruch 1, dadurch gekennzeichnet, daß zur Einstellung der Osmolarität Kochsalz dient.

3. Verwendung nach Anspruch 1 oder 2, dadurch gekennzeichnet, daß der pH-Wert auf 4 bis 8 eingestellt wird.

4. Verwendung nach einem der Ansprüche 1 bis 3, dadurch gekennzeichnet, daß Komplexverbindungen des dreiwertigen Eisens eingesetzt werden.

5. Verwendung nach Anspruch 4, dadurch gekennzeichnet, daß dreiwertiges Eisen als Citratkomplex in einer Menge in Wasser gelöst wird, daß es in der resultierenden Lösung in einer Konzentration von 1 bis 5 mmol/Liter vorliegt.

6. Verwendung nach Anspruch 4, dadurch gekennzeichnet, daß dreiwertiges Eisen als Tartratkomplex in einer Menge in Wasser gelöst wird, daß es in der resultierenden Lösung in einer Konzentration von 0,015 bis 1 mmol/Liter vorliegt.

7. Verwendung nach Anspruch 4, dadurch gekennzeichnet, daß dreiwertiges Eisen in Form eines Eisen-Dextran-Komplexes in einer Menge in Wasser gelöst wird, daß es in der resultierenden Lösung in einer Konzentration von 0,015 bis 1 mmol/Liter vorliegt.

**8.** Verwendung nach einem der Ansprüche 1 bis 3, dadurch gekennzeichnet, daß ein lösliches Salz von Magnesium in einer Menge in Wasser gelöst wird, daß es in der resultierenden Lösung in einer Konzentration von 1 bis 50 mmol/Liter vorliegt.

**Claims**

**Claims for the following Contracting States : AT, BE, CH, DE, FR, GB, IT, LI, LU, NL, SE**

**1.** A solution for use as irrigation fluid, in which in the destruction of exogenous deposits in human or animal tissues or body cavities by means of intense, pulsed laser radiation, especially that in the infrared region, shock waves and cavitation bringing about the destruction are generated by a laser-induced breakthrough, which is composed of an aqueous solution of salts or complex compounds of metals of the iron group of the periodic table, of magnesium or calcium or of mixtures of such salts or complex compounds, which are present in a concentration not exceeding 50 mmol/liter, which is, where appropriate, adjusted by physiologically tolerated, inert additives to an osmolarity which is approximately in the region of that of a physiological saline solution.

**2.** A solution as claimed in claim 1, wherein sodium chloride is used to adjust the osmolarity.

**3.** A solution as claimed in claim 1 or 2, whose pH is from 4 to 8.

**4.** A solution as claimed in any of claims 1 to 3, wherein a complex compound of trivalent iron is used as that of metals of the iron group.

**5.** A solution as claimed in claim 4, which contains trivalent iron as citrate complex in a concentration of from 1 to 5 mmol/liter.

**6.** A solution as claimed in claim 4, which contains trivalent iron as tartrate complex in a concentration of from 0.015 to 1 mmol/liter.

**7.** A solution as claimed in claim 4, which contains trivalent iron in the form of an iron-dextran complex in an Fe-III concentration of from 0.015 to 1 mmol/liter.

**8.** A solution as claimed in any of claims 1 to 3, which contains a soluble salt of magnesium in a concentration of from 1 to 50 mmol/liter.

**Claims for the following Contracting States : ES, GR**

**1.** A solution for use as irrigation fluid, in which in the destruction of exogenous deposits in human or animal tissues or body cavities by means of intense, pulsed laser radiation, especially that in the infrared region, shock waves and cavitation bringing about the destruction are generated by a laser-induced breakthrough, which is composed of an aqueous solution of salts or complex compounds of metals of the iron group of the periodic table, of magnesium or calcium or of mixtures of such salts or complex compounds, which are present in a concentration not exceeding 50 mmol/liter, which is, where appropriate, adjusted by physiologically tolerated, inert additives to an osmolarity which is approximately in the region of that of a physiological saline solution.

**2.** A solution as claimed in claim 1, wherein sodium chloride is used to adjust the osmolarity.

**3.** A solution as claimed in claim 1 or 2, whose pH is from 4 to 8.

**4.** A solution as claimed in any of claims 1 to 3, wherein a complex compound of trivalent iron is used as that of metals of the iron group.

**5.** A solution as claimed in claim 4, which contains trivalent iron as citrate complex in a concentration of from 1 to 5 mmol/liter.

**6.** A solution as claimed in claim 4, which contains trivalent iron as tartrate complex in a concentration of from 0.015 to 1 mmol/liter.

**7.** A solution as claimed in claim 4, which contains trivalent iron in the form of an iron-dextran complex in an Fe-III concentration of from 0.015 to 1 mmol/liter.

**8.** A solution as claimed in any of claims 1 to 3, which contains a soluble salt of magnesium in a concentration of from 1 to 50 mmol/liter.

**Revendications**
**Revendications pour les Etats contractants suivants : AT, BE, CH, DE, FR, GB, IT, LI, LU, NL, SE**

**1.** Solution pour l'utilisation en tant que liquide de rinçage dans lequel, pour la destruction de dépôts étrangers au corps dans les tissus ou cavités du corps humain ou animal, au moyen d'une radiation laser intensive pulsée, en particulier dans le domaine infrarouge, on produit des ondes de choc et une cavitation provoquant la destruction par une rupture induite par laser, caractérisé en ce qu'elle consiste en une solution aqueuse de sels ou complexes de métaux du groupe du fer de la Classification Périodique, du magnésium ou du calcium, ou de mélanges de ces sels ou complexes, présents à une concentration de 50 mmol/l au maximum, qu'on règle le cas échéant au moyen d'additifs inertes acceptables pour l'usage médical à une osmolarité se situant aux environs de celle d'une solution physiologique de chlorure de sodium.

**2.** Solution selon la revendication 1, caractérisée en ce que, pour le réglage de l'osmolarité, on utilise du chlorure de sodium.

**3.** Solution selon la revendication 1 ou 2, caractérisée en ce que son pH est de 4 à 8.

**4.** Solution selon une des revendications 1 à 3, caractérisée en ce que l'on utilise en tant que complexes de métaux du groupe du fer des complexes du fer trivalent.

**5.** Solution selon la revendication 4, caractérisé en ce qu'elle contient le fer trivalent à l'état de complexe citrique à une concentration de 1 à 5 mmol/l.

**6.** Solution selon la revendication 4, caractérisée en ce qu'elle contient le fer trivalent à l'état de complexe tartrique à une concentration de 0,015 à 1 mmol/l.

**7.** Solution selon la revendication 4, caractérisé en ce qu'elle contient le fer trivalent à l'état de complexe fer-dextrane à une concentration en Fe-III de 0,015 à 1 mmol/l.

**8.** Solution selon une des revendications 1 à 3, caractérisée en ce qu'elle contient un sel soluble du magnésium à une concentration de 1 à 50 mmol/l.

**Revendications pour les Etats contractants suivants : ES, GR**

**1.** Utilisation de sels ou complexes de métaux du groupe du fer de la Classification Périodique, du magnésium ou du calcium ou de mélanges de ces sels ou complexes pour la préparation d'un liquide de rinçage aqueux ne contenant que ces sels ou complexes en tant que composés métalliques actifs, pour la destruction de dépôts étrangers dans des tissus ou cavités du corps humain ou animal par des ondes de choc et une cavitation produites dans ce liquide de rinçage par une radiation laser intensive pulsée, en particulier dans le domaine infrarouge, par rupture induite par le laser, ces sels ou complexes étant dissous sous forme d'une solution à une concentration maximale de 50 mmol/l qui, le cas échéant, peut être réglée au moyen d'additifs inertes, acceptables pour l'usage médical, à une osmolarité correspondant à peu près à celle d'une solution physiologique de chlorure de sodium.

**2.** Utilisation selon la revendication 1, caractérisé en ce que pour le réglage de l'osmolarité, on utilise du chlorure de sodium.

**3.** Utilisation selon la revendication 1 ou 2, caractérisée en ce que l'on règle le pH à un niveau de 4 à 8.

**4.** Utilisation selon une des revendications 1 à 3, caractérisée en ce que l'on utilise des complexes du fer trivalent.

**5.** Utilisation selon la revendication 4, caractérisée en ce que l'on dissout du fer trivalent à l'état de complexe citrique dans une quantité d'eau telle que, dans la solution obtenue, il soit à une concentration de 1 à 5 mmol/l.

**6.** Utilisation selon la revendication 4, caractérisée en ce que l'on dissout du fer trivalent à l'état de complexe tartrique dans l'eau en quantité telle que, dans la solution obtenue, il soit à une concentration de 0,015 à 1 mmol/l.

**7.** Utilisation selon la revendication 4, caractérisée en ce que l'on dissout du fer trivalent sous forme de complexe fer-dextrane dans l'eau en quantité telle que dans la solution obtenue, il soit à une concentration de 0,015 à 1 mmol/l.

**8.** Utilisation selon une des revendications 1 à 3, caractérisée en ce que l'on dissout un sel soluble de magnésium dans l'eau en quantité telle que, dans la solution obtenue, il soit à une concentration de 1 à 50 mmol/l.